# EUROPEAN PATENT SPECIFICATION

(11) **EP 1 796 775 B1**
(45) Date of publication and mention of the grant of the patent: **23.06.2010**
(21) Application number: 05779710.2
(22) Date of filing: 09.08.2005
(51) Int. Cl.: A61M 25/09

(54) **POLYMER COATED GUIDE WIRE**
MIT POLYMER ÜBERZOGENER FÜHRUNGSDRAHT
FIL-GUIDE ENDUIT D'UN POLYMERE

(30) Priority: 01.09.2004 US 931746
(43) Date of publication of application: 20.06.2007
(73) Proprietor: Abbott Cardiovascular Systems Inc., Santa Clara, CA 95054 (US)
(72) Inventor: GRANDFIELD, Ryan, Santa Clara, CA 95054-2807 (US)
(74) Representative: McLeish, Nicholas Alistair Maxwell
(86) International application number: PCT/US2005/028101
(87) International publication number: WO 2006/028630

(56) References cited:
- WO-A-01/54761
- WO-A-93/04722
- US-A- 3 973 556
- US-A- 4 534 363
- US-A- 4 538 622
- US-A- 5 341 818
- US-A- 6 042 876

## Description

### BACKGROUND OF THE INVENTION

This invention relates to the field of guide wires for advancing intraluminal devices such as stent delivery catheters, balloon dilatation catheters, atherectomy catheters and the like within a patient's body, specifically, within a patient's vasculature.

In a typical percutaneous procedure in a patient's coronary system, a guiding catheter having a preformed distal tip is percutaneously introduced into a patient's peripheral artery, e.g. femoral, radial or brachial artery, by means of a conventional Seldinger technique and advanced therein until the distal tip of the guiding catheter is seated in the ostium of a desired coronary artery. There are two basic techniques for advancing a guide wire into the desired location within the patient's coronary anatomy, the first is a preload technique which is used primarily for over-the-wire (OTW) devices and the bare wire technique which is used primarily for rail type systems. With the preload technique, a guide wire is positioned within an inner lumen of an OTW device such as a dilatation catheter or stent delivery catheter with the distal tip of the guide wire just proximal to the distal tip of the catheter and then both are advanced through the guiding catheter to the distal end thereof. The guide wire is first advanced out of the distal end of the guiding catheter into the patient's coronary vasculature until the distal end of the guide wire crosses the arterial location where the interventional procedure is to be performed, e.g. a lesion to be dilated or a dilated region where a stent is to be deployed.

The catheter, which is slidably mounted onto the guide wire, is advanced out of the guiding catheter into the patient's coronary anatomy over the previously introduced guide wire until the operative portion of the intravascular device, e.g. the balloon of a dilatation or a stent delivery catheter, is positioned across the arterial location. Once the catheter is in position with the operative means located within the desired arterial location, the interventional procedure is performed. The catheter can then be removed from the patient over the guide wire. Usually, the guide wire is left in place for a period of time after the procedure is completed to ensure reaccess to the arterial location if it is necessary. For example, in the event of arterial blockage due to dissected lining collapse, a rapid exchange type perfusion balloon catheter such as described and claimed in U.S. Patent 5,516,336 (McInnes et al.), can be advanced over the in-place guide wire so that the balloon can be inflated to open up the arterial passageway and allow blood to perfuse through the distal section of the catheter to a distal location until the dissection is reattached to the arterial wall by natural healing.

With the bare wire technique, the guide wire is first advanced by itself through the guiding catheter until the distal tip of the guide wire extends beyond the arterial location where the procedure is to be performed. Then a rail type catheter, such as described in U.S. Patent No. 5,061,273 (Yock) and the previously discussed McInnes et al. is mounted onto the proximal portion of the guide wire which extends out of the proximal end of the guiding catheter which is outside of the patient The catheter is advanced over the guide wire, while the position of the guide wire is fixed, until the operative means on the rail type catheter is disposed within the arterial location where the procedure is to be performed. After the procedure the intravascular device may be withdrawn from the patient over the guide wire or the guide wire advanced further within the coronary anatomy for an additional procedure.

Conventional guide wires for angioplasty, stent delivery, atherectomy and other vascular procedures usually comprise an elongated core member with one or more tapered sections near the distal end thereof and a flexible body such as a helical coil or a tubular body of polymeric material disposed about the distal portion of the core member. A shapeable member, which may be the distal extremity of the core member or a separate shaping ribbon which is secured to the distal extremity of the core member, extends through the flexible body and is secured to the distal end of the flexible body by soldering, brazing or welding which forms a rounded distal tip. Torquing means are provided on the proximal end of the core member to rotate, and thereby steer, the guide wire while it is being advanced through a patient's vascular system.

Specially configured guide wires have been proposed for facilitating the crossing of chronic total occlusions (CTO) or highly occluded sections of vessel. Use of tapered distal tips have been suggested for more easily penetrating the occlusion wherein a variety of structures can be relied upon to provide a strong yet flexible taper. Although a coiled structure delivers strength and flexibility, the coils can be susceptible to becoming snagged on occlusive material and can potentially be subject to being pulled apart upon retraction of the guide wire.

Further details of guide wires, and devices associated therewith for various interventional procedures can be found in U.S. Patent 4,748,986 (Morrison et al.); U.S. Patent 4,538,622 (Samson et al.): U.S. Patent 5,135,503 (Abrams); U.S. Patent 5,341,818 (Abrams et al.); U.S. Patent 5,345,945 (Hodgson, et al.) and U.S. Patent 5,636,641 (Fariabi).

For example, in US Patent No. 6,042,876, which forms basis for the preamble of claim 1, there is described a guide wire comprising a core wire having a coil tip at its distal end. The coil tip includes a helically wound filament having adjacent turns spaced apart by a preselected distance. The guide wire is coated with a polymeric material, typically a hydrophilic polysaccharide, such as hyaluronic acid or chondroitin sulphate. By selecting the spacing between adjacent turns of the coil tip, the hydrophilic coating will adhere to the coil tip in a manner which does not penetrate the coil and which does not significantly interfere with flexibility and bendability of the coil tip.

In US Patent No. 3,973,556 there is described a coil spring guide, for use in connection with the insertion of a catheter into the vessels of a body, that has a coil spring, and a wire core extending within the coil spring and having a distal end welded to the distal end of the coil spring. The coil spring is made from metal wire that in a straightened condition is circular in transverse cross section throughout its length, and that after being coiled, to have adjacent helices abut against one another. Thereafter the radially outer circumferential portion of the coil is ground away to provide a helical radially outer spring coil surface. Advantageously, prior to carrying out the material removal step on the coil spring, the surface of the coil spring is coated with a plastic material. Alternatively the coil spring may be coated with the plastic coating after the material removal step.

While it is most desirable to minimize the force necessary for advancing the guide wire through vasculature it is nonetheless desirable to retain sufficient tactile feedback in order to allow the clinician to feel wire movement. These two performance criteria tend to compete with one another to the extent that an increase in lubricity would typically be expected to result in a decrease in tactile feedback and vice versa. While lubricity and tactile feedback are desirable performance attributes for substantially any guide wire application including cardiovascular, peripheral and carotid procedures, it is especially desirable in total occlusion cases when the guide wire must be sufficiently lubricious to penetrate and navigate tiny micro-channels in the lumen, but nonetheless allow the clinician to feel wire movement A guide wire is needed that is simultaneously capable of satisfying these performance requirements.

### SUMMARY OF THE INVENTION

According to the present invention there is provided a guide wire having the features set out in claim 1.

The guide wire of the present invention overcomes the shortcomings of previously known guide wire configurations to the extent that the device can easily be advanced through vasculature yet provide a high degree of tactile feedback. The guide wire is suitable for use in cardiovascular, peripheral or carotid procedures and is especially well adapted for use in total occlusion cases. The desired performance characteristics are achieved with the application of a polymer to the distal tip of the guide wire that is different to the polymer that is used to coat sections proximal thereto.

In a preferred embodiment, a very thin, highly shape conforming polymer is used to coat the tip coil while a thicker, less conforming polymer is applied proximal thereto. The high conformance of the polymer to the tip coil causes the polymer surface to assume the ribbed shape of the underlying coil. The reduced conformance of the polymer coating proximal to the tip coil serves to present a smoother surface for contacting the vasculature through which it is advanced to thereby greatly reduce drag and resistance. The polymer coatings provide for a lubricious surface to reduce drag and friction while the ribbed surface at the distal tip of the guide wire provides the desired tactile feedback. Additionally, the presence of a polymer between and/or over the coils prevents the coils from becoming snagged on occlusive material and being pulled apart upon retraction of the guide wire.

Specific polymer combinations can be selected from a large variety of polymers so as to achieve the desired performance characteristics of the guide wire of the present invention. Suitable polymers include, but are not limited to, urethanes, nylons, PTFE, and PEP. A polymer that is selected for application to a specific portion of the guide wire may also be loaded with a radiopaque material, such as tungsten, in order to impart a desired degree of lubricity or a desired "feel" to the guide wire in addition to enabling that portion of the guide wire to be visualized during a procedure.

The selected combination ofpolymers can be applied using any of variety of techniques as well as a combination thereof. For example, the guide wire can be dip coated to not only form a polymeric outer surface but to additionally penetrate between coils and fill the space between the coils and an underlying core section. Alternatively, the polymer can be applied in the form of heat shrink tubing or can be extruded there onto. As a further alternative, a coil wire can be coated with the polymer prior to being coiled. The various polymer coatings can be applied so as to overlap, form a butt joint or define a gap there between.

The guide wire of the present invention may be based on any of various guide wire platforms which may in turn incorporate any of the various guide wire components including core tapers, tip flats, and core materials, while tip coil configurations can varied to meet specific design requirement. Typically a guide wire would have an elongate core member with proximal and distal core sections and a flexible tubular body such as a helical coil or polymeric body disposed about and secured to the distal section of the core member. The flexible tubular body such as a helical coil is secured by its distal end to the distal tip of the distal core section or to the distal tip of a shaping ribbon secured to the distal core section in a conventional fashion. The helical coil may be secured at its distal end by application of an adhesive or epoxy, soldering, brazing or welding to form a rounded distal tip to the guiding member as done with commercially available guide wires for procedures within a patient's coronary artery.

These and other advantages of the invention will become apparent from the following derailed description of preferred embodiments which, taken in conjunction with the drawings, illustrate by way of example the principles of the invention.

### BRIEF DESCRIPTION OF THE DRAWINGS

FIG. 1 is a side view of a distal section of a guide wire of the present invention;
FIG. 2 is an enlarged view of the guide wire of FIG. 1;
FIG. 3 is a view similar to FIG. 2 of an alternative embodiment; and
FIG. 4 is a view similar to FIG. 2 of another alternative embodiment of the guide wire of the present invention.

### DETAILED DESCRIPTION OF PREFERRED EMBODIMENTS

The Figures depict various embodiments of the guide wire of the present invention wherein different polymers are applied to selected components of a guide wire in order to modify the performance characteristics of the underlying components. A guide wire can thereby be configured to simultaneously provide for high degree of tactile feedback while being highly lubricious to thereby minimize drag and friction.

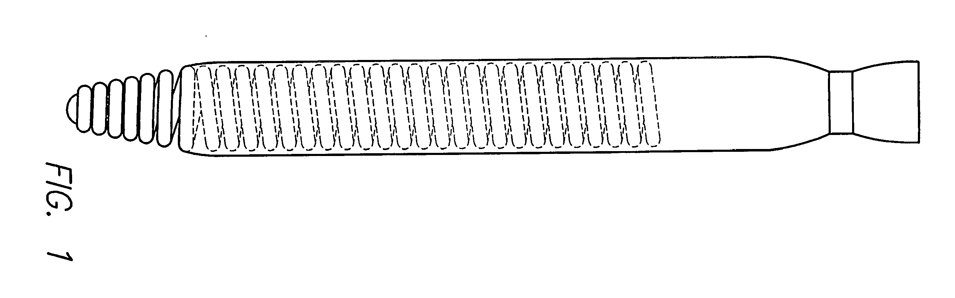

FIG. 1 is a side view of the distal section of guide wire 12 of the present invention that is especially well adapted for use in CTO applications. A tapered distal tip coil 16 is attached to a section of coil of constant diameter 14 which in turn is attached to a core that extends proximally therefrom. A first polymer coating 18 is applied to the distal tip coil wherein such polymer is selected for its ability to conform to an underlying surface and thereby substantially assume the rippled shape of the underlying coil's outer surface. A second different polymer 20 is applied to the coil of constant diameter. Such second polymer is selected for its non conforming properties wherein a substantially smooth outer surface results despite the rippled shape of the exterior surface of the underlying coiled structure. In a preferred embodiment, the highly conforming first polymer extends from the distal tip of the tip coil proximally therefrom while the second polymer extends proximally from a point located approximately 2.5 centimeters form the distal tip.

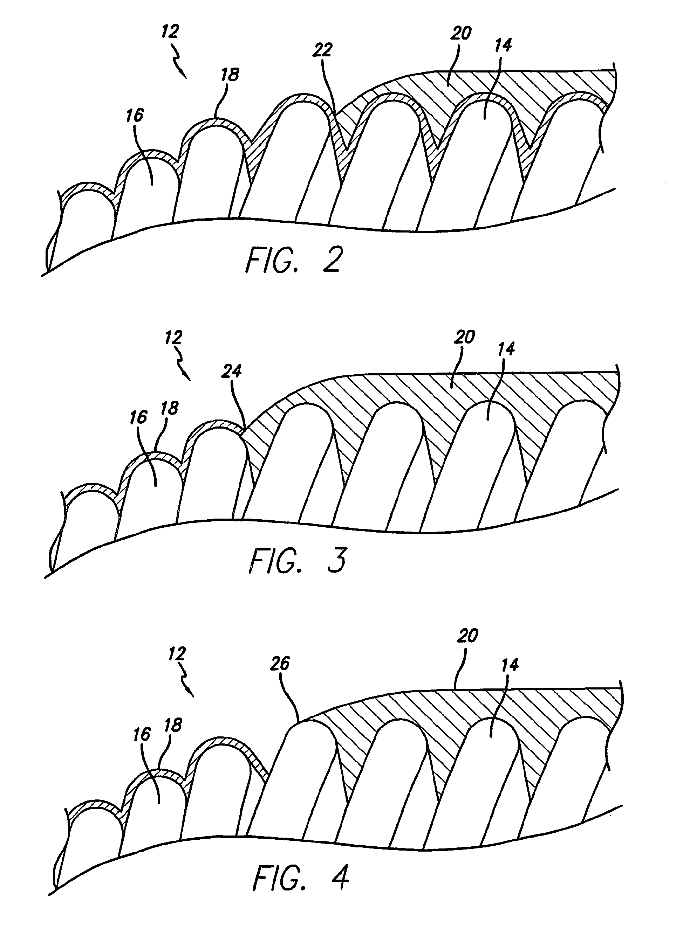

FIG. 2 is an enlarged view of the guide wire 12 shown in FIG.1. This view shows an overlapping configuration of the two polymers 18, 20. The highly conforming first polymer 18 is shown extending from the distal end of the tip coil 16 proximally, while the relatively non-conforming second polymer 20 extends proximally from a point proximal to the proximal end of the distal tip. As result the outer surface of the guide wire has a rippled shape in the tapered section while being smooth proximal thereto. The distal end 22 of the second polymer can substantially coincide with the proximal end of the tip coil or can be positioned along the tip coil or alternatively, at a point along the coil of constant diameter. The overlap between the two polymers may be selected to define a section just a few millimeters in length to a section extending along the entire length of the second polymer.

FIG. 3 is a view similar to FIG. 2 of an alternative embodiment of the guide wire configuration shown in FIG. 2, wherein the first and second polymers 18, 20 form a butt joint 24. The highly conforming first polymer 18 is shown extending proximally from the distal end of the tip coil 16 to a point approximately coinciding with the proximal end of the tip coil. The non conforming second polymer 20 butts up against the proximal end of the first polymer and extends proximally therefrom. Such configuration again provides a structure that includes a distal tip with a rippled surface while a smooth surface extends proximally therefrom. The butt joint can be positioned to coincide with the proximal end of the tapered distal coil, can be positioned along the length of the distal coil or can be positioned along the length of the coil of constant diameter.

FIG. 4 is view similar to FIG. 2 of another alternative embodiment of the guide wire shown in FIG. 2 wherein the proximal end of the first polymer 18 and the distal end of the second polymer 20 are spaced apart from one another so as to define a gap 26. In the particular embodiment that is shown, the gap is positioned just proximal to the proximal end of the distal coil. Such configuration again provides a structure that includes a distal tip with a rippled surface while a smooth surface extends proximally therefrom. The gap can be positioned to coincide with the proximal end of the tapered distal coil, can be positioned along the length of the distal coil or can be positioned along the length of the coil of constant diameter.

A wide variety of polymers can be employed in the practice of the present invention including, but not limited to, urethanes, nylons, PTFE or FEP. The polymer selected for application to the distal most section of the guide wire is selected for its ability to conform to the underlying shape therein properties such as low viscosity and the ability to form a thin layer are critical. The low viscosity also allows the polymer to fill the available space in the interior of the coils as well as bridging the space between adjacent coils. UV curable aliphatic urethane acrylate has been found to be especially well suited for such application. The second polymer is selected for its ability to be applied in a relatively thick layer and for having a sufficiently high viscosity to form a smooth outer surface despite a rippled substrate. A polyurethane ether polymer has been found to be especially well suited for such application.

The polymers applied to the guide wire can be rendered radiopaque in order to enable visualization of the guide wire during a procedure. Either or both of the polymers used to coat the selected sections of the guide wire of the present invention can be loaded with high levels of tungsten in order to achieve the desired degree of visibility.

The selected polymers can be applied to the guide wire using any of a wide variety of methods, including, but not limited to dip coating, spray coating, extrusion, necking or heat shrinking. Well know masking methods can be used to control the application of a polymer to selected portions of the guide wire. The selected polymers can be applied to the coiled structures or the coiled structures can be formed from polymer coated wire.

In use, the guide wire of the present invention offers a desirable degree of tactile feedback to the clinician while simultaneously minimizing drag and friction. The ripples on the leading surfaces of the guide wire serve to briefly engage occlusive material to provide clarity of wire movement while the lubricity of both polymers minimizes drag as the guide wire is advanced especially when negotiating tortuous paths. Additionally, the polymers serve to lock the coils together to prevent them from becoming snagged or from pulling apart as the guide wire is retracted.

While particular forms of the invention have been described and illustrated, it will also be apparent to those skilled in the art that various modifications can be made without departing form the - scope of the invention. Accordingly, it is not intended that the invention be limited except by the appended claims.

## Claims

1. A guide wire (12), comprising:
a distal section (14,16) having a coiled structure wherein its outer surface has a rippled shape; and
a first polymer (18) having high conformance properties applied to a distal portion of said coiled structure wherein its outer surface substantially assumes the rippled shape of the outer surface of the underlying coiled structure, **characterised in that**
said distal section (14,16) of said guide wire (12) includes a tapered tip coil (16) and a coil of constant diameter (14) proximal thereto; and
a second polymer (20) having relatively low conformance properties is applied to a portion of said coiled structure proximal to said distal portion wherein its outer surface does not assume the rippled shape of the underlying coiled structure so as to be substantially smooth.

2. The guide wire (12) of claim 1, wherein said first and second polymers (18,20) overlap one another such that the proximal end of said first polymer (18) is proximal to the distal end of the second polymer (20).

3. The guide wire (12) of claim 1, wherein the proximal end of said first polymer (18) and the distal end of said second polymer (20) are butted up against one another.

4. The guide wire (12) of claim 1, wherein said first and second polymers (18,20) are spaced so as to define a gap (26) between the proximal end of said first polymer (18) and the distal end of said second polymer (20).

5. The guide wire (12) of claim 1, wherein said first polymer (18) comprises a UV curable aliphatic urethane acrylate,

6. The guide wire (12) of claim 1, wherein said second polymer (20) comprises a polyurethane ether polymer.

7. The guide wire (12) of claim 1, wherein said first polymer (18) is radiopaque.

8. The guide wire (12) of claim 1, wherein said second polymer (20) is radiopaque.

9. The guide wire (12) of claim 1, wherein said tapered tip coil (16) substantially comprises said distal portion of said distal section (14,16).

10. The guide wire (12) of claim 1, wherein said first polymer (18) is applied by dip coating.

11. The guide wire (12) of claim 1, wherein at least one of said polymers (18,20) is heat shrunk onto said coiled structure.

12. The guide wire (12) of claim 11, wherein both of said polymers (18,20) are heat shrunk onto said coiled structure.

13. The guide wire (12) of claim 1, wherein said first polymer (18) is applied to wire before such wire is wound to form said coil structure.

14. The guide wire (12) of claim 1, wherein said first polymer (18) is applied to wire before such wire is wound to form said tapered tip coil (16).

15. The guide wire (12) of claim 1, wherein
said first polymer (18) is disposed about said tip coil (16) wherein its outer surface substantially assumes said rippled shape of the outer surface of said tip coil (16); and
said second polymer (20) is disposed about said coil of constant diameter (14) wherein its outer surface is substantially smooth.

## Patentansprüche

1. Führungsdraht (12), umfassend:
einen distalen Abschnitt (14, 16), der eine schraubenförmige Struktur aufweist, wobei seine Außenfläche eine Wellengestalt aufweist; und
ein erstes Polymer (18) von hohem Anpassungsvermögen, welches auf ein distales Teilstück der schraubenförmigen Struktur aufgebracht ist, wobei seine Außenfläche im Wesentlichen die Wellengestalt der Außenfläche der darunterliegenden, schraubenförmigen Struktur annimmt,
**dadurch gekennzeichnet, dass**
der distale Abschnitt (14, 16) des Führungsdrahts (12) eine Wicklung (16) mit sich verjüngender Spitze und eine bezüglich derselben proximal angeordnete Wicklung (14) gleichbleibenden Durchmessers einschließt; und
ein zweites Polymer (20) von relativ niedrigem Anpassungsvermögen auf ein bezüglich des distalen Teilstücks proximal angeordnetes Teilstück der schraubenförmigen Struktur aufgebracht ist, wobei seine Außenfläche die Wellengestalt der darunterliegenden, schraubenförmigen Struktur nicht annimmt, so dass sie im Wesentlichen glatt ist.

2. Führungsdraht (12) gemäß Anspruch 1, bei welchem die ersten und zweiten Polymere (18, 20) einander derart überlappen, dass sich das proximale Ende des ersten Polymers (18) bezüglich des distalen Endes des zweiten Polymers (20) proximal befindet.

3. Führungsdraht (12) gemäß Anspruch 1, bei welchem sich das proximale Ende des ersten Polymers (18) und das distale Ende des zweiten Polymers (20) gegeneinander anlegen.

4. Führungsdraht (12) gemäß Anspruch 1, bei welchem die ersten und zweiten Polymere (18, 20) derart voneinander beabstandet sind, dass zwischen dem proximalen Ende des ersten Polymers (18) und dem distalen Ende des zweiten Polymers (20) ein Spalt (26) festgelegt wird.

5. Führungsdraht (12) gemäß Anspruch 1, bei welchem das erste Polymer (18) ein UV-härtbares, aliphatisches Urethanacrylat umfasst.

6. Führungsdraht (12) gemäß Anspruch 1, bei welchem das zweite Polymer (20) ein Polyurethan-Ether-Polymer umfasst.

7. Führungsdraht (12) gemäß Anspruch 1, bei welchem das erste Polymer (18) strahlenundurchlässig ist.

8. Führungsdraht (12) gemäß Anspruch 1, bei welchem das zweite Polymer (20) strahlenundurchlässig ist.

9. Führungsdraht (12) gemäß Anspruch 1, bei welchem die Wicklung (16) mit sich verjüngender Spitze im Wesentlichen das distale Teilstück des distalen Abschnitts (14, 16) umfasst.

10. Führungsdraht (12) gemäß Anspruch 1, bei welchem das erste Polymer (18) mittels Tauchbeschichten aufgebracht ist.

11. Führungsdraht (12) gemäß Anspruch 1, bei welchem wenigstens eines der Polymere (18, 20) mittels Wärmeschrumpfen auf die schraubenförmige Struktur aufgebracht ist.

12. Führungsdraht (12) gemäß Anspruch 11, bei welchem beide Polymere (18, 20) mittels Wärmeschrumpfen auf die schraubenförmige Struktur aufgebracht sind.

13. Führungsdraht (12) gemäß Anspruch 1, bei welchem das erste Polymer (18) auf den Draht aufgebracht ist, bevor dieser Draht gewickelt wird, um die schraubenförmige Struktur zu bilden.

14. Führungsdraht (12) gemäß Anspruch 1, bei welchem das erste Polymer (18) auf den Draht aufgebracht ist, bevor dieser Draht gewickelt wird, um die Wicklung (16) mit sich verjüngender Spitze zu bilden.

15. Führungsdraht (12) gemäß Anspruch 1, bei welchem
das erste Polymer (18) um die Spitzenwicklung (16) herum angeordnet ist, wobei seine Außenfläche im Wesentlichen die Wellengestalt der Außenfläche der Spitzenwicklung (16) annimmt; und
das zweite Polymer (20) um die Wicklung (14) gleichbleibenden Durchmessers herum angeordnet ist, wobei seine Außenfläche im Wesentlichen glatt ist.

## Revendications

1. Fil de guidage (12), comprenant :
une section distale (14, 16) ayant une structure spiralée où sa surface externe a une forme ondulée ; et
un premier polymère (18) ayant des propriétés de conformité élevées appliqué à une partie distale de ladite structure spiralée où sa surface externe prend essentiellement la forme ondulée de la surface externe de la structure spiralée sous-jacente, **caractérisé en ce que**
ladite section distale (14, 16) dudit fil de guidage (12) comporte une bobine (16) de pointe conique et une bobine à diamètre constant (14) qui lui est proximale ; et
un deuxième polymère (20) ayant des propriétés de conformité relativement faibles est appliqué à une partie de ladite structure spiralée proximale à ladite partie distale où sa surface externe ne prend pas la forme ondulée de la structure spiralée sous-jacente de sorte à être essentiellement lisse.

2. Fil de guidage (12) de la revendication 1, dans lequel lesdits premier et deuxième polymères (18, 20) se chevauchent de sorte que l'extrémité proximale dudit premier polymère (18) soit proximale à l'extrémité distale du deuxième polymère (20).

3. Fil de guidage (12) de la revendication 1, dans lequel l'extrémité proximale dudit premier polymère (18) et l'extrémité distale dudit deuxième polymère (20) sont en butée l'une contre l'autre.

4. Fil de guidage (12) de la revendication 1, dans lequel lesdits premier et deuxième polymères (18, 20) sont espacés de sorte à définir un espace (26) entre l'extrémité proximale dudit premier polymère (18) et l'extrémité distale dudit deuxième polymère (20).

5. Fil de guidage (12) de la revendication 1, dans lequel ledit premier polymère (18) comprend un acrylate d'uréthane aliphatique durcissable aux UV.

6. Fil de guidage (12) de la revendication 1, dans lequel ledit deuxième polymère (20) comprend un polymère polyuréthanne éther.

7. Fil de guidage (12) de la revendication 1, dans lequel ledit premier polymère est radio-opaque.

8. Fil de guidage (12) de la revendication 1, dans lequel ledit deuxième polymère (20) est radio-opaque.

9. Fil de guidage (12) de la revendication 1, dans lequel ladite bobine (16) de pointe conique comprend essentiellement ladite partie distale de ladite section (14, 16) distale.

10. Fil de guidage (12) de la revendication 1, dans lequel ledit premier polymère est appliqué par revêtement par immersion.

11. Fil de guidage (12) de la revendication 1, dans lequel au moins l'un desdits polymères (18, 20) est thermorétracté sur ladite structure spiralée.

12. Fil de guidage (12) de la revendication 11, dans lequel lesdits deux polymères (18, 20) sont thermorétractés sur ladite structure spiralée.

13. Fil de guidage (12) de la revendication 1, dans lequel ledit premier polymère (8) est appliqué à un fil avant qu'un tel fil ne soit enroulé pour former ladite structure spiralée.

14. Fil de guidage (12) de la revendication 1, dans lequel ledit premier polymère (8) est appliqué à un fil avant qu'un tel fil ne soit enroulé pour former ladite bobine (16) de pointe conique.

15. Fil de guidage (12) de la revendication 1, dans lequel
ledit premier polymère (18) est disposé autour de ladite bobine (16) de pointe où sa surface externe prend essentiellement ladite forme ondulée de la surface externe de ladite bobine (16) de pointe; et
ledit deuxième polymère (20) est disposé autour de ladite bobine de diamètre constant (14) où sa surface externe est essentiellement lisse.
